(19) **Europäisches Patentamt / European Patent Office / Office européen des brevets**

(11) **EP 2 663 864 B1**

(12) # EUROPEAN PATENT SPECIFICATION

(45) Date of publication and mention of the grant of the patent:
**10.04.2019 Bulletin 2019/15**

(21) Application number: **12734581.7**

(22) Date of filing: **17.01.2012**

(51) Int Cl.:
*C12Q 1/686* (2018.01)   *C12Q 1/6869* (2018.01)

(86) International application number:
**PCT/US2012/021594**

(87) International publication number:
**WO 2012/097374 (19.07.2012 Gazette 2012/29)**

(54) **IMMUNODIVERSITY ASSESSMENT METHOD AND ITS USE**

IMMUNODIVERSITÄTS-SCHNELLBESTIMMUNGSVERFAHREN UND SEINE ANWENDUNG

PROCÉDÉ D'ÉVALUATION DE LA BIODIVERSITÉ ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.01.2011 US 201161432638 P**

(43) Date of publication of application:
**20.11.2013 Bulletin 2013/47**

(73) Proprietor: **CB Biotechnologies, Inc.**
**Huntsville, Alabama 35802 (US)**

(72) Inventor: **HAN, Jian**
**Huntsville**
**Alabama 35802 (US)**

(74) Representative: **Dehns**
**St. Brides House**
**10 Salisbury Square**
**London EC4Y 8JD (GB)**

(56) References cited:
WO-A1-2011/139371   WO-A2-2004/033728
US-A1- 2006 160 153   US-A1- 2010 021 896
US-A1- 2010 330 571   US-A1- 2011 003 291

- SCOTT D BOYD ET AL: "Measurement and clinical monitoring of human lymphocyte clonality by massively parallel VDJ pyrosequencing", SCIENCE TRANSLATIONAL MEDICINE, vol. 1, no. 12, 23 December 2009 (2009-12-23), XP055076874,
- GEORGE B. COHEN ET AL: "Clonotype Tracking of TCR Repertoires during Chronic Virus Infections", VIROLOGY, vol. 304, no. 2, 1 December 2002 (2002-12-01), pages 474-484, XP55275967, AMSTERDAM, NL ISSN: 0042-6822, DOI: 10.1006/viro.2002.1743
- WANG ET AL.: 'High throughput sequencing reveals a complex pattern of dynamic interrelationships among human T cell subsets.' PROC NATL ACAD SCI USA vol. 107, no. 4, 26 January 2010, pages 1518 - 1523, XP055114155
- LANGERAK A W ET AL: "Molecular and flow cytometric analysis of the Vbeta repertoire for clonality assessment in mature TCRalphabeta T-cell proliferations", BLOOD, AMERICAN SOCIETY OF HEMATOLOGY, US, vol. 98, no. 1, 1 July 2001 (2001-07-01), pages 165-173, XP002287368, ISSN: 0006-4971, DOI: 10.1182/BLOOD.V98.1.165

**EP 2 663 864 B1**

**Description**

This application claims the benefit of priority of United States Provisional Application Number 61/432,638, filed January 14, 2011.

The invention relates to methods for the assessment of the level of diversity in an immunorepertoire.

A single diagnostic test rarely exists for the definitive diagnosis of an individual disease. However, diagnostic tests may be used to detect the presence or absence of the normal state and the results of these tests may be used to screen patients and collectively aid in diagnosis. For example, a normal white blood cell count is between 4,500 and 10,000 cells per microliter. An elevated white blood cell count is not determinative for a specific disease, but it may indicate an underlying problem that requires medical evaluation. Normal ranges of red blood cell counts for women and men are generally different, with a count of 5 to 6 million per microliter being normal for males and 3.6 to 5.6 million being normal for females. Platelet counts are normal if they are within the range of 150,000 to 400,000. In the presence of inflammation, for example, red cell count may go down, white cell count may go up, and platelet count may also be elevated.

Blood glucose levels may be used as early indicators associated with diseases as varied as Cushing syndrome, hyperthyroidism, pancreatic cancer, pancreatitis, pre-diabetes, and diabetes. Heart disease, the tendency to have heart disease, signs of certain cancers, and a variety of genetic diseases may have as their early signs one or more abnormal results for a variety of diagnostic tests. Diagnostic tests which individually and/or collectively indicate normal health or the absence of normal health (*i.e.*, disease) include, for example, measures of albumin, alkaline phosphatase, alanine transaminase (ALT), aspartate aminotransferase (AST), blood urea nitrogen (BUN), serum calcium, serum chloride, carbon dioxide, creatinine, direct bilirubin, gamma-glutamyl-transpeptidase (gamma-GT), glucose, lactate dehydrogenase (LDH), serum phosphorus, potassium, serum sodium, total bilirubin, total cholesterol, total protein, and uric acid.

Disease prevention also requires a more precise description of normal status, because only when we can better describe what is normal can we identify deviations from normal or subnormal, and evaluate the effectiveness of preventive measures.

Although many diagnostic tests are currently available and performed on a regular basis, many diseases, such as cancer and heart disease, may go undetected until they have progressed to the point where clear physical symptoms are present. Far too often, it is too late at that point to provide effective treatment. Therefore, there is always a need for additional tests which may be used to assess the health of a patient and to identify abnormal states that may signal the presence of serious disease or the predisposition to such disease.

Boyd et al. (Science Translational Medicine, 1 (12), 2009) discloses the measurement and clinical monitoring of human lymphocyte clonality by massively parallel VDJ pyrosequencing.

Cohen et al. (Virology, 304 (2), 2002) discloses clonotype tracking of TCR repertoires during chronic virus infections.

WO 2004/033728 discloses PCR-based clonality studies for early diagnosis of lymphoproliferative disorders and detection of minimal residual disease.

US 2011/0003291 discloses a method for analysing the diversity of the catalogue of T and/or B lymphocytes in an individual, based on the amplification, from a sample, of genomic DNA fragments by PCR multi-n-plexes, with n ≥ 2, carried out with a combination of at least 3 primers defining at least 2 primer couples, each of which includes a primer specifically hybridising upstream and/or in a given V or D gene and a primer specifically hybridising downstream and/or in a given J gene, in order to obtain the amplificiation of at least two fragments characteristic of two distinct V-J or D-J rearrangements from each primer couple.

Langerak et al. (Blood, 98 (1), 2001) discloses the molecular and flow cytometric analysis of the Vbeta repertoire for clonality assessment in mature TCRalphabeta T-cell proliferations.

US 2010/330571 discloses a method of measuring immunocompetence which provides a means for assessing the effects of diseases or conditions that compromise the immune system and of therapies aimed to reconstitute it which is based on quantifying T-cell diversity by calculating the number of diverse T-cell receptor (TCR) beta chain variable regions from blood cells.

Disclosed herein is a method for identifying a normal immune status or an abnormal immune status in an individual, a normal immune status being indicated by the presence of a diverse target population of detectable immune system cells and an abnormal immune status being indicated by the lack of such diversity. The method comprises quantifying clonotypes of immune system cells and identifying the number of clonotypes which comprise a significant percentage of a total number of cells counted within that population. The normal state is characterized by the presence of a greater variety (or diversity) of clonotypes represented by the significant percentage of the total number of cells and an abnormal state is characterized by the presence of a significantly lower number of clonotypes represented by the significant percentage of the total number of cells. For example, the most important region of the TCR is the third complementarity-determining region (CDR3) whose nucleotide sequence is unique to each T cell clone. The significant percentage may be a number from about 25 to about 75 percent. In various aspects of the invention, the inventor has found that a significant percentage of fifty percent (50%) provides a useful diagnostic result. Where the "significant percentage" of the total number cells is fifty percent (50%), the diversity index (D50) is a measure of the diversity of an immune repertoire of J individual cells (the total number of CDR3s) composed of S distinct CDR3s in a ranked dominance configuration where $r_i$ is the abundance of the $i_{th}$ most abundant CDR3, $r_1$ is the abundance of the most abundant CDR3, $r_2$ is the abundance of the second most abundant CDR3, and so on. C is the minimum number of distinct CDR3s, amounting to ≥50% of the total sequencing reads. D50 therefore is given by C/S x 100.

$$Assume\ that\ \underbrace{r_1 \geq r_2 \cdots \geq r_i \geq r_{i+1} \cdots \geq r_S}_{S}, \sum_{i=1}^{S} r_i = J$$

$$if\ \sum_{i=1}^{C} r_i \geq J/2\ and\ \sum_{i=1}^{C-1} r_i < J/2$$

$$D50 = \frac{C}{S} \times 100$$

In various aspects of the invention, the immune system cells that are quantified may include, for example, all T cells [panT], functional subsets of T cells such as $CD8^+$ T cells [cytotoxic T ($T_c$)] $CD4^+$ T cells and their subsets [$T_H1$, $T_H2$, $T_H17$, regulatory T ($T_{reg}$) and follicular T ($T_{FH}$)], or developmental subsets of T cells such as naive T ($T_n$), activated T ($T_a$), memory T ($T_m$), all B cells (panB) and their subsets such as naive B ($B_n$), activated B ($B_a$), memory B ($B_m$), plasma and plasmablast B cells. In various aspects of the invention, the significant percentage may be any value from about 25% to about 75%. In some aspects, the significant percentage may be 50%.

The invention relates to a method for assessing the level of diversity of an immunorepertoire, the method comprising the steps of (a) amplifying polynucleotides from a population of white blood cells from a human or animal subject in a reaction mix comprising target-specific nested primers to produce a set of first amplicons, at least a portion of the target-specific nested primers comprising additional nucleotides which, during amplification, serve as a template for incorporating into the first amplicons a binding site for at least one common primer; (b) transferring a portion of the first reaction mix containing the first amplicons to a second reaction mix comprising at least one common primer; (c) amplifying, using the at least one common primer, the first amplicons to produce a set of second amplicons; (d) sequencing the second amplicons to identify V(D)J rearrangement sequences in the subpopulation of white blood cells, and (e) using the identified V(D)J rearrangement sequences to quantify both the total number of cells in a population of immune system cells and the total numbers of cells within each distinct CDR3 clonotype identified within the population; and (f) identifying the number of distinct CDR3 clonotypes that comprise a significant percentage (N) of a total number of cells counted within that population, wherein a normal state is characterized by the presence of a greater variety of distinct CDR3 clonotypes represented within the significant percentage (N) of the total number of cells and an abnormal state is characterized by the presence of a lesser number of distinct CDR3 clonotypes represented within significant percentage (N) of the total number of cells, wherein the diversity of the distinct CDR3 clonotypes is measured by the diversity index ($D_N$), wherein $D_N$ is determined by C/S x 100, where C is a minimum number of distinct CDR3 clonotypes amounting to greater than or equal to N percent of a total of sequencing reads obtained following amplification and sequencing of polynucleotides isolated from the population of cells, wherein S is the number of distinct CDR3 clonotypes in ranked dominance configuration.

The disclosure also has application in respect to evaluating microbial diversity. Shifts in microbial populations and population numbers have been noted in obesity, in diabetes, and in inflammatory conditions of the intestine, for example. Identifying normal and abnormal diversity profiles using the method of the invention may be useful as a diagnostic test using clinical samples taken from nasal passages, oral cavities, skin, the gastrointestinal tract, and/or urogenital tract, for example.

Brief Description of the Drawings

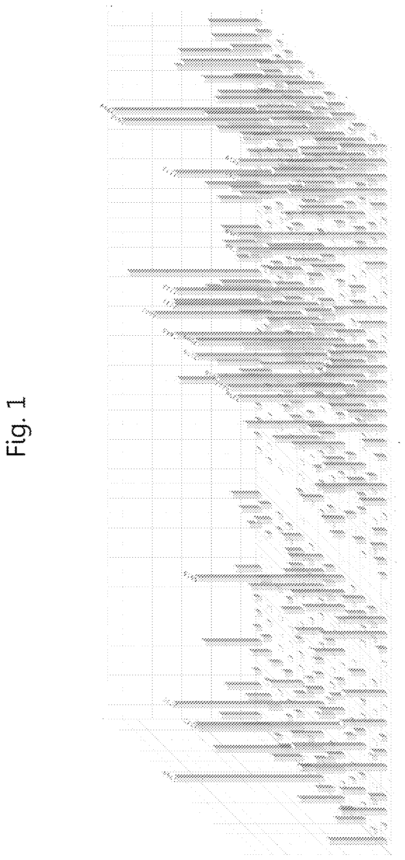

Fig. 1 is a graph illustrating the relative numbers of T-cell clonotypes in the blood of a normal individual.

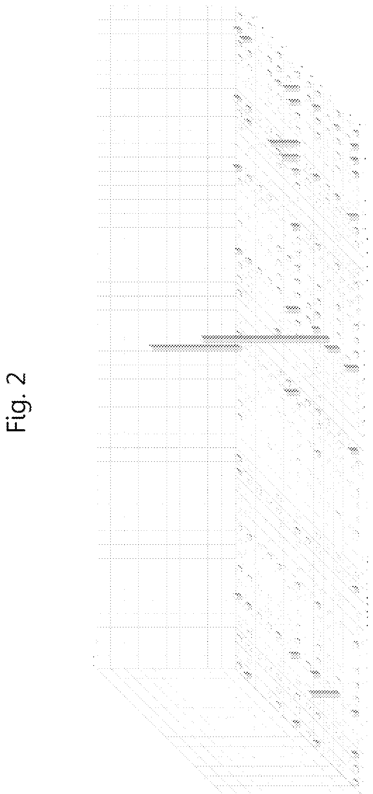

Fig. 2 is a graph illustrating the relative numbers of T-cell clonotypes in the blood of an individual who has been diagnosed with colon cancer. Two clones clearly stand out, with these clones having been expanded to a level where they constitute a significant percentage of the total number of clonotypes.

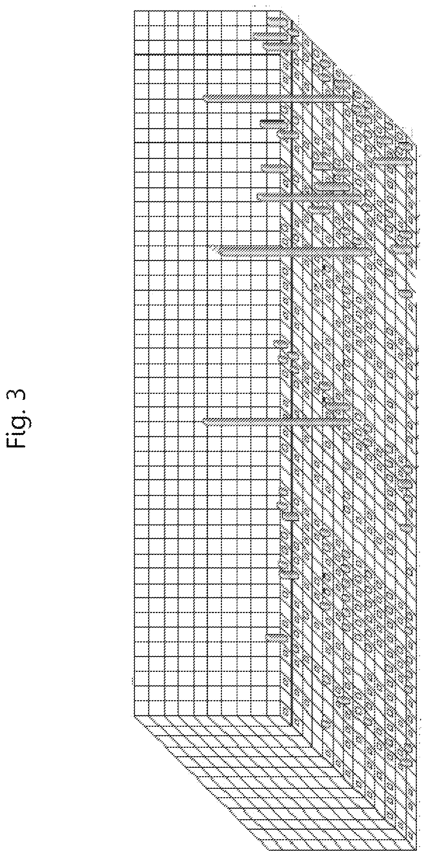

Fig. 3 is a graph illustrating the relative numbers of T-cell clonotypes in the blood of an individual who has been diagnosed with breast cancer (cytotoxic T cell subset). Four clones clearly stand out, making up a significant percentage of the total number of clonotypes.

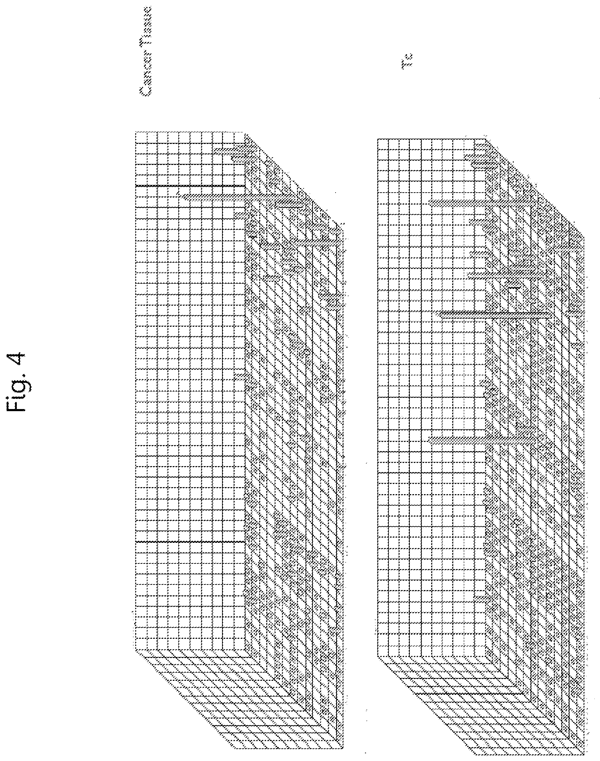

Fig.4 shows two graphs (4a and 4b), with 4a representing the relative numbers of T-cell clonotypes detected in a sample taken from cancer tissue, while 4b represents the total numbers of T-cell clonotypes detected in a sample taken from the blood of the same patient (cytotoxic T cell subset). The dominant clone comprising the differential clonal expansion of the immunorepertoire of this patient is from the Tc subset.

Detailed Description

The inventor has developed a method for distinguishing the immunorepertoires of normal, healthy individuals from those of individuals who have symptomatic and/or non-symptomatic disease. This method uses the difference between the level of immune cell diversity generally seen in a normal, healthy individual and the generally lower level of diversity seen in an individual who has one or more disease conditions as a diagnostic indicator of the presence of a normal or abnormal immune status. In one aspect of the invention, the diversity level is referred to as the D50, with D50 being defined as the minimum percentage of distinct CDR3s accounting for at least half of the total CDR3s in a population or subpopulation of immune system cells. The third complementarity-determining region (CDR3) being a region whose nucleotide sequence is unique to each T or B cell clone, the higher the number, the greater the level of diversity. D50 may be described as follows. Where the "significant percentage" of the total number cells is fifty percent (50%), the diversity index (D50) may also be defined as a measure of the diversity of an immune repertoire of J individual cells (the total number of CDR3s) composed of S distinct CDR3s in a ranked dominance configuration where $r_i$ is the abundance of the ith most abundant CDR3, $r_1$ is the abundance of the most abundant CDR3, $r_2$ is the abundance of the second most abundant CDR3, and so on. C is the minimum number of distinct CDR3s, amounting to 50% of the total sequencing reads. D50 therefore is given by C/S x 100.

$$\textit{Assume that } \underbrace{r_1 \geq r_2 \cdots \geq r_i \geq r_{i+1} \cdots \geq r_S}_{S}, \sum_{i=1}^{S} r_i = J$$

$$\textit{if } \sum_{i=1}^{C} r_i \geq J/2 \textit{ and } \sum_{i=1}^{C-1} r_i < J/2$$

$$D50 = \frac{C}{S} \times 100$$

The method of the invention is performed using the following steps for assessing the level of diversity of an immunorepertoire: (a) amplifying polynucleotides from a population of white blood cells from a human or animal subject in a reaction mix comprising target-specific nested primers to produce a set of first amplicons, at least a portion of the target-specific nested primers comprising additional nucleotides which, during amplification, serve as a template for incorporating into the first amplicons a binding site for at least one common primer; (b) transferring a portion of the first reaction mix containing the first amplicons to a second reaction mix comprising at least one common primer; (c) amplifying, using the at least one common primer, the first amplicons to produce a set of second amplicons; (d) sequencing the second amplicons to identify V(D)J rearrangement sequences in the subpopulation of white blood cells, (e) using the identified V(D)J rearrangement sequences to quantify both the total number of cells in a population of immune system cells and the total numbers of cells within each of the clonotypes identified within the population; and (f) identifying the number of clonotypes that comprise a significant percentage of a total number of cells counted within that population, wherein a normal state is characterized by the presence of a greater variety of clonotypes represented within the significant percentage (N) of the total number of cells and an abnormal state is characterized by the presence of a lesser number of clonotypes represented within a significant percentage (N) of the total number of cells, wherein the diversity of the clonotypes is measured by the diversity index ($D_N$), wherein $D_N$ is determined by C/S x 100, where C is a minimum number of distinct clonotypes amounting to greater than or equal to N percent of a total of S sequencing reads obtained following amplification and sequencing of polynucleotides isolated from the population of cells.

It has previously been difficult to assess the immune system in a broad manner, because the number and variety of cells in a human or animal immune system is so large that sequencing of more than a small subset of cells has been almost impossible. The inventor developed a semi-quantitative PCR method (arm-PCR, described in more detail in U.S. Patent Application Publication Number 20090253183), which provides increased sensitivity and specificity over previously-available methods, while producing semi-quantitative results. It is this ability to increase specificity and sensitivity, and thereby increase the number of targets detectable within a single sample that makes the method ideal for detecting relative numbers of clonotypes of the immunorepertoire. The inventor has more recently discovered that using this sequencing method allows him to compare immunorepertoires of individual subjects, which has led to the development of the present method. The method has been used to evaluate subjects who appear normal, healthy, and asymptomatic, as well as subjects who have been diagnosed with various forms of cancer, for example, and the inventor has demonstrated that the presence of disease correlates with decreased immunorepertoire diversity, which can be readily detected using the method of the invention. This method may therefore be useful as a diagnostic indicator, much as cell counts and biochemical tests are currently used in clinical practice.

Clonotypes (*i.e.,* clonal types) of an immunorepertoire are determined by the rearrangement of Variable(V), Diverse(D) and Joining(J) gene segments through somatic recombination in the early stages of immunoglobulin(Ig) and T cell receptor (TCR) production of the immune system. The V(D)J rearrangement can be amplified and detected from T cell receptor alpha, beta, gamma, and delta chains, as well as from immunoglobulin heavy chain (IgH) and light chains (IgK, IgL). Cells may be obtained from a patient by obtaining peripheral blood, lymphoid tissue, cancer tissue, or tissue or fluids from other organs and/or organ systems, for example. Techniques for obtaining these samples, such as blood samples, are known to those of skill in the art. "Quantifying clonotypes," as used herein, means counting, or obtaining a reliable approximation of, the numbers of cells belonging to a particular clonotype. Cell counts may be extrapolated from the number of sequences detected by PCR amplification and sequencing.

The CDR3 region, comprising about 30-90 nucleotides, encompasses the junction of the recombined variable (V), diversity (D) and joining (J) segments of the gene. It encodes the binding specificity of the receptor and is useful as a sequence tag to identify unique V(D)J rearrangements.

Wang *et al.* disclosed that PCR may be used to obtain quantitative or semi-quantitative assessments of the numbers of target molecules in a specimen (Wang, M. et al., "Quantitation of mRNA by the polymerase chain reaction," (1989) Proc. Nat'l. Acad. Sci. 86: 9717-9721). Particularly effective methods for achieving quantitative amplification have been described previously by the inventor. One such method is known as arm-PCR, which is described in United States Patent Application Publication Number 20090253183A1.

Aspects of the invention include arm-PCR amplification of CDR3 from T cells, B cells, and/or subsets of T or B cells. The term "population" of cells, as used herein, therefore encompasses what are generally referred to as either "populations" or "subpopulations" of cells. Large numbers of amplified products may then be efficiently sequenced using next-generation sequencing using platforms such as 454 or Illumina, for example. If the significant percentage that is chosen is 50%, the number may be referred to as the "D50." D50 may then be the percent of dominant and unique T or B cell clones that account for fifty percent (50%) of the total T or B cells counted in that sample. For high-throughput sequencing, for example, the D50 may be the number of the most dominant CDR3s, among all unique CDR3s, that make up 50% of the total effective reads, where total effective reads is defined as the number of sequences with identifiable V and J gene segments which have been successfully screened through a series of error filters.

The arm-PCR method provides highly sensitive, semi-quantitative amplification of multiple polynucleotides in one reaction. The arm-PCR method may also be performed by automated methods in a closed cassette system (iCubate®, Huntsville, Alabama), which is beneficial in the present method because the repertoires of various T and B cells, for example, are so large. In the arm-PCR method, target numbers are increased in a reaction driven by DNA polymerase, which is the result of target-specific primers being introduced into the reaction. An additional result of this amplification reaction is the introduction of binding sites for common primers which will be used in a subsequent amplification by transferring a portion of the first reaction mix containing the first set of amplicons to a second reaction mix comprising common primers. "At least one common primer," as used herein, refers to at least one primer that will bind to such a binding site, and includes pairs of primers, such as forward and reverse primers. This transfer may be performed either by recovering a portion of the reaction mix from the first amplification reaction and introducing that sample into a second reaction tube or chamber, or by removing a portion of the liquid from the completed first amplification, leaving behind a portion, and adding fresh reagents into the tube in which the first amplification was performed. In either case, additional buffers, polymerase, etc., may then be added in conjunction with the common primers to produce amplified products for detection. The amplification of target molecules using common primers gives a semi-quantitative result wherein the quantitative numbers of targets amplified in the first amplification are amplified using common, rather than target-specific primers -making it possible to produce significantly higher numbers of targets for detection and to determine the relative amounts of the cells comprising various rearrangements within a patient blood sample. Also, combining the second reaction mix with a portion of the first reaction mix allows for higher concentrations of target-specific primers to be added to the first reaction mix, resulting in greater sensitivity in the first amplification reaction. It is the combination of specificity and sensitivity, along with the ability to achieve quantitative results by use of a method such as the arm-PCR method, that allows a sufficiently sensitive and quantitative assessment of the type and number of clonotypes in a population of cells to produce a diversity index that is of diagnostic use.

Clonal expansion due to recognition of antigen results in a larger population of cells that recognize that antigen, and evaluating cells by their relative numbers provides a method for determining whether an antigen exposure has influenced expansion of antibody-producing B cells or receptor-bearing T cells. This is helpful for evaluating whether there may be a particular population of cells that is prevalent in individuals who have been diagnosed with a particular disease, for example, and may be especially helpful in evaluating whether or not a vaccine has achieved the desired immune response in individuals to whom the vaccine has been given.

Primers for amplifying and sequencing variable regions of immune system cells are available commercially, and have been described in publication such as the inventor's published patent applications WO2009137255 and US201000021896A1.

There are several commercially available high-throughput sequencing technologies, such as Hoffman-LaRoche, Inc.'s 454® sequencing system. In the 454® sequencing method, for example, the A and B adaptor are linked onto PCR products either during PCR or ligated on after the PCR reaction. The adaptors are used for amplification and sequencing steps. When done in conjunction with the arm-PCR technique, A and B adaptors may be used as common primers (which are sometimes referred to as "communal primers" or "superprimers") in the amplification reactions. After A and B adaptors have been physically attached to a sample library (such as PCR amplicons), a single-stranded DNA library is prepared using techniques known to those of skill in the art. The single-stranded DNA library is immobilized onto specifically-designed DNA capture beads. Each bead carries a unique singled-stranded DNA library fragment. The bead-bound library is emulsified with amplification reagents in a water-in-oil mixture, producing microreactors, each containing just one bead with one unique sample-library fragment. Each unique sample library fragment is amplified within its own microreactor, excluding competing or contaminating sequences. Amplification of the entire fragment collection is done in parallel. For each fragment, this results in copy numbers of several million per bead. Subsequently, the emulsion PCR is broken while the amplified fragments remain bound to their specific beads. The clonally amplified fragments are enriched and loaded onto a PicoTiterPlate® device for sequencing. The diameter of the PicoTiterPlate® wells allows for only one bead per well. After addition of sequencing enzymes, the fluidics subsystem of the sequencing instrument flows individual nucleotides in a fixed order across the hundreds of thousands of wells each containing a single bead. Addition of one (or more) nucleotide(s) complementary to the template strand results in a chemilluminescent signal recorded by a CCD camera within the instrument. The combination of signal intensity and positional information generated across the PicoTiterPlate® device allows the software to determine the sequence of more than 1,000,000 individual reads, each is up to about 450 base pairs, with the GS FLX system.

Having obtained the sequences using a quantitative and/or semi-quantitative method, it is then possible to calculate the D50, for example, by determining the percent of clones that account for at least about 50% of the total clones detected in the patient sample. Normal ranges may be compared to the numbers obtained for an individual patient, and the result may be reported both as a number and as a normal or abnormal result. This provides a physician with an additional clinical test for diagnostic purposes. Results for patient samples from a healthy individual, a patient with colon cancer, and a patient with lung cancer are shown below in Table 1. These results are from T-cell populations, expressed as an average of results from 8 (age matched normal) to 10 (colon cancer, lung cancer) samples.

**Table 1**

| Health Condition | D50(Tc) | D50(Tr) | D50(Th) |
|---|---|---|---|
| Healthy/Normal | 23.6 | 43.5 | 38.9 |
| Colon Cancer | 4.5 | 21.7 | 28.3 |
| Lung Cancer | 4.5 | 17.1 | 26.8 |

As each number represents the percent of clones making up about 50 percent of the total number of sequences detected in the population being assessed, it is clear from the numbers above that a lack of immunorepertoire diversity, expressed as a deviation from normal, may be a useful criterion for use in diagnostic test panels. The method of the invention, particularly if used in an automated system such as that described by the inventor in U.S. Patent Application Publication Number 201000291668A1, may be used to analyze samples from multiple patients, with detection of the amplified targets sequences being accomplished by the use of one or more microarrays.

Hybridization, utilizing at least one microarray, may also be used to determine the D50 of an individual's immunorepertoire. In such a method, the D50 would be calculated as the percentage of the most dominant variable genes (V and/or J genes) which would account for at least 50% of the total signal from all the V and or J genes.

Table 2 illustrates the difference in B-cell diversity, as evidenced by the D50, between (8) normal, healthy individual and (20) individuals with chronic lymphocytic leukemia, and (12) Lupus patients

**Table 2**

| Patient Condition | D50(IgH) |
|---|---|
| Healthy/Normal | 95.3 |
| Chronic Lymphocytic Leukemia | 17.86 |
| Lupus | 26.5 |

Recently, researchers in various laboratories have reported that microbial diversity within a human or animal (the "microbiome") also shifts when the healthy state changes to a more unhealthy state. For example, shifts in microbial populations have been associated with various gastrointestinal disorders, with obesity, and with diabetes, for example. Zaura *et al*. (Zaura, E. et al. "Defining the healthy 'core microbiome' of oral microbial communities." BMC Microbiology (2009) 9: 259) reported that a major proportion of bacterial sequences of unrelated healthy individuals is identical, and the proportion shifts in individuals who have oral disease. The arm-PCR method, combined with high-throughput sequencing, provides a relatively fast, highly sensitive, specific, and semi-quantitative method for evaluating diversity of microbial populations to establish a microbial D50 value, for example, for various human or animal tissues. Arm-PCR has been shown to be quite effective for identifying bacteria within mixed populations obtained from clinical samples.

Examples

Patient Samples.

Whole blood samples (40 ml) collected in sodium heparin from 10 lung and 10 colon, and 10 breast cancer patients were purchased from Conversant Healthcare Systems (Huntsville, Alabama). Whole blood samples (40 ml) collected in sodium heparin from 8 normal control samples were purchased from ProMedDx (Norton, MA).

*Isolation of T cell subsets.*

T cell isolations were performed using superparamagnetic polystyrene beads (MiltenyiBiotec) coated with monoclonal antibodies specific for each T cell subset. From whole blood, mononuclear cells were obtained by Ficoll prep, and monocytes removed using anti-CD14 microbeads. This monocyte-depleted mononuclear fraction was then used as a source for specific T cell subset fractions.
Cytotoxic CD8+ T cells were isolated by negative selection using anti-CD4 multisort beads (MiltenyiBiotec), followed by positive selection with anti-CD8 beads. CD4+ T cells were isolated by positive selection with anti-CD4 beads. Anti-CD25 beads (MiltenyiBiotec) were used to select CD4+CD25+ regulatory T cells. All isolated cell populations were immediately resuspended in RNAprotect (Qiagen).

*RNA extraction and repertoire amplification*

RNA extraction was performed using the RNeasy Mini Kit (Qiagen) according to the manufacturer's protocol. For each target, a set of nested sequence-specific primers (Forward-out, Fo; Forward-in, Fi; Reverse-out, Ro; and Reverse-in, Ri) was designed using primer software available at www.irepertoire.com. A pair of common sequence tags was linked to all internal primers (Fi and Ri). Once these tag sequences were incorporated into the PCR products in the first few amplification cycles, the exponential phase of the amplification was carried out with a pair of communal primers. In the first round of amplification, only sequence-specific nested primers were used. The nested primers were then removed by exonuclease digestion and the first-round PCR products were used as templates for a second round of amplification by adding communal primers and a mixture of fresh enzyme and dNTP. Each distinct barcode tag was introduced into amplicon from the same sample through PCR primer.

*Sequencing*

Barcode tagged amplicon products from different samples were pooled together and loaded into a 2% agarose gel. Following electrophoresis, DNA fragments were purified from DNA band corresponding to 250-500bp fragments extracted from agarose gel. DNA was sequenced using the 454 GS FLX system with titanium kits (SeqWright, Inc.).

*Sequencing data analysis*

Sequences for each sample were sorted out according to barcode tag. Following sequence separation, sequence analysis was performed in a manner similar to the approach reported by Wang et al. (Wang C, et al. High throughput sequencing reveals a complex pattern of dynamic interrelationships among human T cell subsets. Proc Natl Acad Sci USA 107(4): 1518-1523). Briefly, germline V and J reference sequences, which were downloaded from the IMGT server (http://www.imgt.org), were mapped onto sequence reads using the program IRmap. The boundaries defining CDR3 region in reference sequences were mirrored onto sequencing reads through mapping information. The enclosed CDR3 regions in sequencing reads were extracted and translated into amino acid sequence.

## Claims

1. A method for assessing the level of diversity of an immunorepertoire comprising the steps of:

     (a) amplifying polynucleotides from a population of white blood cells from a human or animal subject in a reaction mix comprising target-specific nested primers to produce a set of first amplicons, at least a portion of the target-specific nested primers comprising additional nucleotides which, during amplification, serve as a template for incorporating into the first amplicons a binding site for at least one common primer;
     (b) transferring a portion of the first reaction mix containing the first amplicons to a second reaction mix comprising at least one common primer;
     (c) amplifying, using the at least one common primer, the first amplicons to produce a set of second amplicons;
     (d) sequencing the second amplicons to identify V(D)J rearrangement sequences in the subpopulation of white blood cells;
     (e) using the identified V(D)J rearrangement sequences to quantify both the total number of cells in a population of immune system cells and the total numbers of cells within each distinct CDR3 clonotype identified within the population; and
     (f) identifying the number of distinct CDR3 clonotypes that comprise a significant percentage (N) of a total number of cells counted within that population, wherein a normal state is **characterized by** the presence of a greater variety of distinct CDR3 clonotypes represented within the significant percentage (N) of the total number of cells and an abnormal state is **characterized by** the presence of a lesser number of distinct CDR3 clonotypes represented within significant percentage (N) of the total number of cells,

     wherein the diversity of the distinct CDR3 clonotypes is measured by the diversity index ($D_N$), wherein $D_N$ is determined by C/S x 100, where C is a minimum number of distinct CDR3 clonotypes amounting to greater than or equal to N percent of a total of sequencing reads obtained following amplification and sequencing of polynucleotides isolated from the population of cells, wherein S is the number of distinct CDR3 clonotypes in ranked dominance configuration.

2. The method of claim 1, wherein the significant percentage (N) is a percentage number from 25 to 75.

3. The method of claim 1, wherein the significant percentage (N) is about 50 percent.

## Patentansprüche

1. Verfahren zur Bestimmung des Diversitätsgrads eines Immunrepertoires, umfassend die folgenden Schritte:

     (a) Amplifizieren von Polynucleotiden aus einer Population von weißen Blutkörperchen aus einem menschlichen oder tierischen Subjekt in einem Reaktionsgemisch, das zielspezifische verschachtelte Primer umfasst, um einen Satz erster Amplikons zu erzeugen, wobei mindestens ein Teil der zielspezifischen verschachtelten Primer zusätzliche Nukleotide umfasst, die, während der Amplifikation, als eine Matrize für den Einbau einer Bindungsstelle für mindestens einen gemeinsamen Primer in die ersten Amplikons dienen;
     (b) Übertragen eines Teils des ersten Reaktionsgemisches, das die ersten Amplikons enthält, in ein zweites Reaktionsgemisch, das mindestens einen gemeinsamen Primer umfasst;
     (c) Amplifizieren, unter Verwendung des mindestens einen gemeinsamen Primers, der ersten Amplikons, um einen Satz zweiter Amplikons zu erzeugen;
     (d) Sequenzieren der zweiten Amplikons, um V(D)J-Umlagerungssequenzen in der Teilpopulation von weißen Blutkörperchen zu identifizieren;

(e) Verwenden der identifizierten V(D)J-Umlagerungssequenzen, um sowohl die Gesamtzahl der Zellen in einer Population von Immunsystemzellen als auch die Gesamtzahl von Zellen innerhalb jedes einzelnen unterschiedlichen CDR3-Klonotyps zu quantifizieren, der innerhalb der Population identifiziert wurde; und

(f) Identifizieren der Anzahl unterschiedlicher CDR3-Klonotypen, die einen signifikanten Prozentsatz (N) einer Gesamtzahl von Zellen umfasst, die innerhalb dieser Population gezählt werden, wobei ein normaler Zustand durch die Gegenwart einer größeren Varietät unterschiedlicher CDR3-Klonotypen gekennzeichnet ist, die innerhalb des signifikanten Prozentsatzes (N) der Gesamtzahl von Zellen dargestellt wird und wobei ein anormaler Zustand durch die Gegenwart einer geringeren Anzahl unterschiedlicher CDR3-Klonotypen gekennzeichnet ist, die innerhalb eines signifikanten Prozentsatzes (N) der Gesamtzahl von Zellen dargestellt ist,

wobei die Diversität der unterschiedlichen CDR3-Klonotypen durch den Diversitätsindex ($D_N$) gemessen wird, wobei $D_N$ durch $C/S \times 100$ bestimmt wird, wo C eine minimale Anzahl unterschiedlicher CDR3-Klonotypen ist, die größer oder gleich N Prozent einer Gesamtzahl von Sequenzierungslesevorgängen ist, die nach Amplifikation und Sequenzierung von Polynucleotiden erhalten werden, die aus der Zellpopulation isoliert wurden, wobei S die Anzahl unterschiedlicher CDR3-Klonotypen in einer gestuften Dominanz-Konfiguration ist.

2. Verfahren nach Anspruch 1, wobei der signifikante Prozentsatz (N) eine Prozentsatzzahl von 25 bis 75 ist.

3. Verfahren nach Anspruch 1, wobei der signifikante Prozentsatz (N) etwa 50 Prozent beträgt.

## Revendications

1. Procédé d'évaluation du niveau de diversité d'un immunorépertoire comprenant les étapes consistant à :

(a) amplifier des polynucléotides d'une population de globules blancs venant d'un sujet humain ou animal dans un mélange réactionnel comprenant des amorces imbriquées spécifiques à la cible pour produire un ensemble de premiers amplicons, au moins une partie des amorces imbriquées spécifiques à la cible comprenant des nucléotides supplémentaires qui, au cours de l'amplification, servent de modèle pour incorporer aux premiers amplicons un site de liaison pour au moins une amorce commune ;

(b) transférer une partie du premier mélange réactionnel contenant les premiers amplicons à un second mélange réactionnel comprenant au moins une amorce commune ;

(c) amplifier, en utilisant la au moins une amorce commune, les premiers amplicons pour produire un ensemble de seconds amplicons ;

(d) séquencer les seconds amplicons pour identifier des séquences de réaménagement V(D)J dans la sous-population de globules blancs ;

(e) utiliser les séquences de réaménagement V(D)J identifiées pour quantifier à la fois le nombre total de cellules dans une population de cellules du système immunitaire et les nombres totaux de cellules dans chaque clonotype CDR3 distinct identifié dans la population ; et

(f) identifier le nombre de clonotypes CDR3 distincts qui comprennent un pourcentage significatif (N) d'un nombre total de cellules comptées dans cette population, dans lequel un état normal est **caractérisé par** la présence d'une plus grande variété de clonotypes CDR3 distincts représentés dans le pourcentage significatif (N) du nombre total de cellules et un état anormal est **caractérisé par** la présence d'un moindre nombre de clonotypes CDR3 distincts représentés dans le pourcentage significatif (N) du nombre total de cellules,

dans lequel la diversité des clonotypes CDR3 distincts est mesurée par l'indice de diversité ($D_N$), dans lequel $D_N$ est déterminé par $C/S \times 100$, où C est un nombre minimal de clonotypes CDR3 distincts se montant à un nombre supérieur ou égal à N pour cent d'un total de lectures de séquençage obtenues après amplification et séquençage de polynucléotides isolés de la population de cellules, dans lequel S est le nombre de clonotypes CDR3 distincts en configuration de dominance classée.

2. Procédé selon la revendication 1, dans lequel le pourcentage significatif (N) est un nombre en pourcentage de 25 à 75.

3. Procédé selon la revendication 1, dans lequel le pourcentage significatif (N) est d'environ 50 pour cent.

Fig. 1

Fig. 2

Fig. 3

Fig. 4

Cancer Tissue

Tc

EP 2 663 864 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61432638 B
- WO 2004033728 A
- US 20110003291 A
- US 2010330571 A
- US 20090253183 A
- US 20090253183 A1
- WO 2009137255 A
- US 201000021896 A1
- US 201000291668 A1

**Non-patent literature cited in the description**

- **BOYD et al.** *Science Translational Medicine,* 2009, vol. 1 (12
- **COHEN et al.** *Virology,* 2002, vol. 304 (2
- **LANGERAK et al.** *Blood,* 2001, vol. 98 (1
- **WANG, M. et al.** Quantitation of mRNA by the polymerase chain reaction. *Proc. Nat'l. Acad. Sci,* 1989, vol. 86, 9717-9721
- **ZAURA, E. et al.** Defining the healthy 'core microbiome' of oral microbial communities. *BMC Microbiology,* 2009, vol. 9, 259
- **WANG C et al.** High throughput sequencing reveals a complex pattern of dynamic interrelationships among human T cell subsets. *Proc Natl Acad Sci USA,* vol. 107 (4), 1518-1523